# EUROPEAN PATENT APPLICATION

(11) **EP 1 002 865 A1**
(43) Date of publication of application: **24.05.2000**
(21) Application number: 98402726.8
(22) Date of filing: 30.10.1998
(51) Int. Cl.: C12N 15/17, C12Q 1/68, C07K 14/575, C07K 16/26, A61K 38/22

(54) **Adipose specific protein**

(71) Applicant: Sanofi-Synthélabo, 75013 Paris (FR)
(72) Inventor: Fraser, Robert, 92500 Rueil Malmaison (FR); Angel, Itzchak, Nes-Ziyyona (IL); Guillot, Etienne, 91190 Gif sur Yvette (FR)
(74) Representative: Desaix, Anne

(57) **Abstract**

ASP-1 polypeptides and polynucleotides and methods for producing such polypeptides by recombinant techniques are disclosed. Also disclosed are methods for utilizing ASP-1 polypeptides and polynucleotides in the design of protocols for the treatment of obesity, cardiovascular disorders such as hypertension and arteriosclerosis, diabetes, hypo- and hyperglycemia and anorexia, among others, and diagnostic assays for such conditions.

SEQUENCE LISTING

(1) GENERAL INFORMATION:

(i) APPLICANT:
(A) NAME: SYNTHELABO
(B) STREET: 22 AVENUE GALILEE
(C) CITY: LE PLESSIS
(E) COUNTRY: FRANCE
(F) POSTAL CODE (ZIP): 92350

(ii) TITLE OF INVENTION: ADIPOSE SPECIFIC PROTEIN

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 1605 base pairs
(B) TYPE: nucleic acid
(C) STRANDEDNESS: single
(D) TOPOLOGY: circular

(ii) MOLECULE TYPE: DNA (genomic)

(iii) HYPOTHETICAL: NO

(iv) ANTI-SENSE: NO

(v) FRAGMENT TYPE: N-terminal

(xi) SEQUENCE DESCRIPTION: SEQ ID NO: 1:

CCGATTCATT AATGCAGCTG GCAGACAGTT TCCCGAATGG AAAGCGGGCA GTGAGCGCAC 60

GCAATTAATG TGAGTTAGCT CACTCATTAG GCACCCCAGG CTTTACACTT TATGCTTCCG 120

GCTCGTATGT TGTGTGGAAT TGTGAGCGGA TAACAATTTC ACACAGGAAA CAGCTATGAC 180

CATGATTACG CCAAGCTCGA AATTAACCCT CACTAAAGGG AACAAAAGCT GGAGCTCCAC 240

CGCGGTGGCG GCCGCTCTAG AACTAGTGGA TCCCCCGGGC TGCAGGAATT CGGCACGAGC 300

CAGGAGGCTG TGAGGGGGAG AATGTTCTTT TGGCCACTGT GAAGCCTCAG GAAGGGGCTC 360

GGATTGCTCA AGGACCCATG GGAGAGAGGA GGCTTTGACT GGGCTGCCTG CCTGTGAGGT 420

CTCTGGACTA GAGGTCCAAC GCAGTCCAGC TGACAAGGAT GGAATACGCC ATGAAGTCCC 480

TTAGCCTTCT GTACCCCAAG TCCCTCTCCA GGCATGTGTC AGTGCGTACC TCTGTGGTGA 540

CCCAGCAGCT GCTGTCGGAG CCCAGCCCCA AGGCCCCCAG GGCCCGGCCC TGCCGCGTAA 600

GCACGGCGGA TCGAAGCGTG AGGAAGGGCA TCATGGCTTA CAGTCTTGAG GACCTCCTCC 660

TCAAGGTCCG GGACACTCTG ATGCTGGCAG ACAAGCCCTT CTTCCTGGTG CTGGAGGAAG 720

ATGGCACAAC TGTAGAGACA GAAGAGTACT TCCAAGCCCT GGCAGGGGAT ACAGTGTTCA 780

TGGTCCTCCA GAAGGGGCAG AAATGGCAGC CCCCATCAGA ACAGGGGACA AGGCACCCAC 840

TGTCCCTCTC CCATAAGCCT GCCAAGAAGA TTGATGTGGC CCGTGTAACG TTTGATCTGT 900

ACAAGCTGAA CCCACAGGAC TTCATTGGCT GCCTGAACGT GAAGGCGACT TTTTATGATA 960

CATACTCCCT TTCCTATGAT CTGCACTGCT GTGGGGCCAA GCGCATCATG AAGGAAGCTT 1020

TCCGCTGGGC CCTCTTCAGC ATGCAGGCCA CAGGCCACGT ACTGCTTGGC ACCTCCTGTT 1080

ACCTGCAGCA GCTCCTCGAT GCTACGGAGG AAGGGCAGCC CCCCAAGGGC AAGGCCTCAT 1140

CCCTTATCCC GACCTGTCTG AAGATACTGC AGTGAAAGCC CAAGTCCTTG GAAGCTTTCC    1200

CCAGTGAAGG ACTGACTGGG GGCCTCACGC TTAACTGGTA GTGCCCACAA GCCTGGCAGC    1260

TGTAGAGCCG CGAACCTCCC CACACCTCCC TCACCGCGCA GGACCCTGAG TGAGGAGGAG    1320

GAGCTGGAAA CCTGGGGTGG GTTGGCCAAA GGAGAACCTC AAGCTCCTGG CCTGATCCAG    1380

CTCCTTCCTG CCCAAGGCAG CTTAGCCCAT CCAGACTGGT CCTGAAGTCT GTCCCTCCAT    1440

TGGCATGAAG TCTGCCCCTT AGCAATCCGG CCTCGCAGGC TGTACTTTCA TGGTGCTCTC    1500

TACCTTCTGG CCCCCATCCC GGAACATTCC TGAGTGAATT CGCAAGCGCA CTAGCATGTG    1560

ATATTAGGGA GTTTGCAATA AATTATTGAG GCTGAAAAAA AAAAA                    1605

(2) INFORMATION FOR SEQ ID NO: 2:

(i) SEQUENCE CHARACTERISTICS:
(A) LENGTH: 238 amino acids
(B) TYPE: amino acid
(C) STRANDEDNESS:
(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(xi) SEQUENCE DESCRIPTION: SEQ ID NO: 2:

Met Glu Tyr Ala Met Lys Ser Leu Ser Leu Leu Tyr Pro Lys Ser Leu
1               5                   10                  15

Ser Arg His Val Ser Val Arg Thr Ser Val Val Thr Gln Gln Leu Leu
            20                  25                  30

Ser Glu Pro Ser Pro Lys Ala Pro Arg Ala Arg Pro Cys Arg Val Ser
        35                  40                  45
Thr Ala Asp Arg Ser Val Arg Lys Gly Ile Met Ala Tyr Ser Leu Glu
    50                  55                  60
Asp Leu Leu Leu Lys Val Arg Asp Thr Leu Met Leu Ala Asp Lys Pro
65                  70                  75                  80
Phe Phe Leu Val Leu Glu Glu Asp Gly Thr Thr Val Glu Thr Glu Glu
                85                  90                  95
Tyr Phe Gln Ala Leu Ala Gly Asp Thr Val Phe Met Val Leu Gln Lys
            100                 105                 110
Gly Gln Lys Trp Gln Pro Pro Ser Glu Gln Gly Thr Arg His Pro Leu
        115                 120                 125
Ser Leu Ser His Lys Pro Ala Lys Lys Ile Asp Val Ala Arg Val Thr
    130                 135                 140
Phe Asp Leu Tyr Lys Leu Asn Pro Gln Asp Phe Ile Gly Cys Leu Asn
145                 150                 155                 160
Val Lys Ala Thr Phe Tyr Asp Thr Tyr Ser Leu Ser Tyr Asp Leu His
                165                 170                 175
Cys Cys Gly Ala Lys Arg Ile Met Lys Glu Ala Phe Arg Trp Ala Leu
            180                 185                 190
Phe Ser Met Gln Ala Thr Gly His Val Leu Leu Gly Thr Ser Cys Tyr
        195                 200                 205
Leu Gln Gln Leu Leu Asp Ala Thr Glu Glu Gly Gln Pro Pro Lys Gly
    210                 215                 220
Lys Ala Ser Ser Leu Ile Pro Thr Cys Leu Lys Ile Leu Gln
225                 230                 235

## Description

The present invention relates to newly identified polynucleotides, polypeptides encoded by them and to the use of such polynucleotides and polypeptides, and to their production. More particularly, the polynucleotides and polypeptides of the present invention relate to the adipose specific protein family, hereinafter referred to as Adipose Specific Protein 1 (ASP-1). The invention also relates to inhibiting or activating the action of such polynucleotides and polypeptides.

Figures from the NIH indicate that half of the population in the United States are overweight and one-third are clinically obese, weighing at least 20% more than they should. Those individuals who remain obese are at high risk for diabetes and cardiovascular diseases, including hypertension, possibly leading to premature death.. Obesity results from a long term positive imbalance of energy intake over expenditure. A biological perspective on obesity regards such imbalances as reflecting dysregulations of energy intake and expenditure that are in turn informed by genetic differences between the obese and the non-obese. Although most human obesity does not follow a Mendalian inheritance pattern , adoption and twin studies indicate strong genetic influence on body fat content. Therefore, analysis of the specific expression of genes in adipose tissue can give important insights into the mechanisms contributing to the imbalances resulting in obesity. Genes with adipose specific expression can be prime targets for the diagnosis and treatment of obesity.

The adipose specific protein described in this invention shares sequence similarity with a known mouse fat specific protein, FSP27. This protein is found only in differentiated adipocytes. Although FSP27 appears to be regulated by the known adipocyte differentiating transcription factor C/EBP, its function in the differentiated adipocyte is currently unknown. However, ASP-1 protein sequence exhibits similarity with a known factor involved in apoptosis, the DNA fragmentation factor (DFF). Apoptosis, refers to the process of programmed cell loss that is desirable for the survival of the host maintaining a balance between cell division and cell loss. Certain pathologies have been associated with a dysregulation of apoptosis; malignant growth associated with cancer (neoplasia), inflammation of tissues and tissue hypertrophy. A cascade of multiple signalling pathways lead from death-triggering extracellular agents to a central control and execution. The physical characteristics associated with apoptosis, which distinguish it from other forms of cell death, are membrane blebbing (zeosis), nuclear collapse, chromatin condensation and marginalisation in crescents around the nuclear envelope, plasma membrane bound apoptitic body formation (Note: plasma membrane remains intact as it still excludes vital dyes and retains most of its internal contents) and phagocytosis of apoptitic cells or bodies prior to their lysis. Under such controlled conditions, unwanted cells can be eliminated without damage to neighbouring cells. Thus, regulation of apoptosis in obese individuals offers the potential of removing unwanted adipose tissue without harm to other cells in the body. Based on the sequence similarity with DFF, ASP-1 can be considered a putative regulator of apoptosis, specifically in adipose tissue. Thus, targeting specific mechanisms controlling apoptosis in adipose tissue may alleviate obesity as well as the elevated risk factors associated with it.

This indicates that the adipose specific protein family has an established, proven history as therapeutic targets. Clearly there is a need for identification and characterization of further members of the adipose specific protein family which can play a role in preventing, ameliorating or correcting dysfunctions or diseases, including, but not limited to, obesity, cardiovascular disorders such as hypertension and arteriosclerosis, diabetes, hypo- and hyperglycemia and anorexia.

In one aspect, the invention relates to ASP-1 polypeptides and recombinant materials and methods for their production. Another aspect of the invention relates to methods for using such ASP-1 polypeptides and polynucleotides. Such uses include the treatment of obesity, cardiovascular disorders such as hypertension and arteriosclerosis, diabetes, hypo- and hyperglycemia and anorexia, among others. In still another aspect, the invention relates to methods to identify agonists and antagonists using the materials provided by the invention, and treating conditions associated with ASP-1 imbalance with the identified compounds. Yet another aspect of the invention relates to diagnostic assays for detecting diseases associated with inappropriate ASP-1 activity or levels.

The following definitions are provided to facilitate understanding of certain terms used frequently herein.

"ASP-1" refers, among others, generally to a polypeptide having the amino acid sequence set forth in SEQ ID NO:2 or an allelic variant thereof.

"ASP-1 activity or ASP-1 polypeptide activity" or "biological activity of the ASP-1 or ASP-1 polypeptide" refers to the metabolic or physiologic function of said ASP-1 including similar activities or improved activities or these activities with decreased undesirable side-effects. Also included are antigenic and immunogenic activities of said ASP-1.

"ASP-1 gene" refers to a polynucleotide having the nucleotide sequence set forth in SEQ ID NO:1 or allelic variants thereof and/or their complements.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al.,* "Analysis for protein modifications and nonprotein cofactors", *Meth Enzymol* (1990) 182:626-646 and Rattan *et al.,* "Protein Synthesis: Posttranslational Modifications and Aging", *Ann NY Acad Sci* (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide without modifying the function of said polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCS program package (Devereux, J., *et al., Nucleic Acids Research* (1984) 12(1):387), BLASTP, BLASTN, FASTA (Atschul, S.F. *et al., J Molec Biol* (1990) 215:403).

As an illustration, by a polynucleotide having a nucleotide sequence having at least, for example, 95% "identity" to a reference nucleotide sequence of SEQ ID NO: 1 is intended that the nucleotide sequence of the polynucleotide is identical to the reference sequence except that the polynucleotide sequence may include up to five point mutations per each 100 nucleotides of the reference nucleotide sequence of SEQ ID NO: 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO:2 is intended that the amino acid sequence of the polypeptide is identical to the reference sequence except that the polypeptide sequence may include up to five amino acid alterations per each 100 amino acids of the reference amino acid of SEQ ID NO: 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Polypeptides according to the present Invention

In one aspect, the present invention relates to ASP-1 polypeptides. The ASP-1 polypeptides include the polypeptide of SEQ ID NO:2 as well as polypeptides comprising the amino acid sequence of SEQ ID NO: 2 and polypeptides comprising the amino acid sequence which have at least 80% identity to that of SEQ ID NO:2 over its entire length, and still more preferably at least 90% identity, and even still more preferably at least 95% identity to SEQ ID NO: 2. Furthermore, those with at least 97-99% are highly preferred. Also included within ASP-1 polypeptides are polypeptides having the amino acid sequence which have at least 80% identity to the polypeptide having the amino acid sequence of SEQ ID NO:2 over its entire length, and still more preferably at least 90% identity, and still more preferably at least 95% identity to SEQ ID NO:2. Furthermore, those with at least 97-99% are highly preferred. Preferably ASP-1 polypeptide exhibit at least one biological activity of ASP-1.

The ASP-1 polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the ASP-1 polypeptides are also included in the invention.

Fragments of ASP-1 polypeptide are characterized in that:
a) they retain the biological activity of ASP-1 including antigenic activity or,
b) they have the sequence of ASP-1 polypeptides of claim 10 or 11 deleted of a continuous series of residues including the amino terminus or the carboxyl terminus or both, or
c) they are a variant of a) or b) by conservative amino-acid substitutions.

More generally, a fragment is a polypeptide having an amino acid sequence that entirely is the same as part, but not all, of the amino acid sequence of the aforementioned ASP-1 polypeptides. As with ASP-1 polypeptides, fragments may be "free-standing," or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of ASP-1 polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of ASP-1 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate ASP-1 activity, including those with a similar activity or an improved activity, or with a decreased undesirable activity. Also included are those that are antigenic or immunogenic in an animal, especially in a human.

Preferably, all of these polypeptide fragments retain the biological activity of the ASP-1, including antigenic activity.

Variants of the defined sequence and fragments also form part of the present invention. Preferred variants are those that vary from the referents by conservative amino acid substitutions - i.e., those that substitute a residue with another of like characteristics. Typical such substitutions are among Ala, Val, Leu and Ile ; among Ser and Thr ; among the acidic residues Asp and Glu ; among Asn and Gln ; and among the basic residues Lys and Arg ; or aromatic residues Phe and Tyr. Particularly preferred are variants in which several, 5-10, 1-5, or 1-2 amino acids are substituted, deleted, or added in any combination.

The ASP-1 polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides according to the present Invention

Another aspect of the invention relates to ASP-1 polynucleotides. ASP-1 polynucleotides include isolated polynucleotides which encode the ASP-1 polypeptides and fragments, and polynucleotides closely related thereto. More specifically, ASP-1 polynucleotide of the invention include a polynucleotide comprising the nucleotide sequence set forth in SEQ ID NO:1 encoding a ASP-1 polypeptide of SEQ ID NO: 2, and polynucleotide having the particular sequence of SEQ ID NO:1. ASP-1 polynucleotides further include a polynucleotide comprising a nucleotide sequence that has at least 80% identity to a nucleotide sequence encoding the ASP-1 polypeptide of SEQ ID NO:2 over its entire length, and a polynucleotide that is at least 80% identical to that having SEQ ID NO:1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under ASP-1 polynucleotides are a nucleotide sequence which has sufficient identity to a nucleotide sequence contained in SEQ ID NO:1 to hybridize under conditions useable for amplification or for use as a probe or marker. The invention also provides polynucleotides which are complementary to such ASP-1 polynucleotidos.

Also included are fragments of ASP-1 polynucleotides having at least 80% identity to SEQ ID NO:2 or complementary to said nucleotide sequence, and encoding biologically active polypeptides such as defined hereabove.

In particular, the present invention provides an isolated human polynucleotide comprising a nucleotide sequence which is complementary to a nucleotide sequence that has at least 80% identity to a nucleotide sequence encoding the ASP-1 polypeptide of SEQ ID NO:2 over its entire length.

Preferably such polynucleotide is DNA or RNA.

ASP-1 of the invention is structurally related to other proteins of the adipose specific protein family, as shown by the results of sequencing the cDNA encoding human ASP-1 and shown in SEQ ID NO:1. The cDNA sequence of SEQ ID NO:1 contains an open reading frame (nucleotide number 459 to 1175) encoding a polypeptide of 238 amino acids of SEQ ID NO:2. Amino acid sequence of SEQ ID NO:2 has about 80% identity (using Lipman-Pearson Protein Alignment) in 1 to 166 amino acid residues with mouse FSP27 (accession number A42445) Nucleotide sequence of SEQ ID NO:1 has about 82% identity (using Wibur-Lipman DNA Alignment) in 303 to 590 nucleotide residues with rat FSP27 (Danesch et al. J. Biol. Chem., 267: 7185, 1992).

One polynucleotide of the present invention encoding ASP-1 may be obtained using standard cloning and screening, from a cDNA library derived from mRNA in cells of human adipose using the expressed sequence tag (EST) analysis (Adams, M.D., *et al. Science* (1991) 252:1651-1656; Adams, M.D. *et al., Nature,* (1992) 355:632-634; Adams, M.D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding ASP-1 polypeptide of SEQ ID NO:2 may be identical to the polypeptide encoding sequence contained in 459 to 1175 of SEQ ID NO:1, or it may be a sequence, which as a result of the redundancy (degeneracy) of the genetic code, also encodes the polypeptide of SEQ ID NO:2.

When the polynucleotides of the invention are used for the recombinant production of ASP-1 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al., Proc Natl Acad Sci USA* (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding ASP-1 variants comprise the amino acid sequence ASP-1 polypeptide of SEQ ID NO:2 in which several, 5-10, 1-5, 1-3, 1-2 or 1 amino acid residues are substituted, deleted or added, in any combination.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

Polynucleotides of the invention, which are identical or sufficiently identical to a nucleotide sequence contained in SEQ ID NO:1 or a fragment thereof, may be used as hybridization probes for cDNA or genomic DNA, to isolate or amplify full-length cDNAs and genomic clones encoding ASP-1 polypeptide and to isolate cDNA and genomic clones of other genes that have a high sequence similarity to the ASP-1 gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding ASP-1 polypeptide comprises the steps of screening an appropriate library under stingent hybridization conditions with a labeled probe having the SEQ ID NO: 1 or a fragment thereof; and isolating full-length cDNA and genomic clones containing said polynucleotide sequence. Such hybridization techniques are well known to those of skill in the art. Stringent hybridization conditions are as defined above or alternatively conditions under overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

### Vectors, Host Cells, Expression according to the present Invention

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *BASIC METHODS IN MOLECULAR BIOLOGY* (1986) and Sambrook et al., *MOLECULAR CLONING: A LABORATORY MANUAL*, 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used.

Thus the present invention also provides a DNA or RNA molecule comprising an expression system, wherein said expression system is capable of producing a ASP-1 polypeptide comprising an amino acid sequence, which has at least 80% identity with the polypeptide of SEQ ID NO:2 when said expression system is present in a compatible host cell.

The appropriate nucleotide sequence may be inserted into such an expression system by any of a variety of well-known and routine techniques, such as, for example, those set forth in Sambrook *et al., MOLECULAR CLONING, A LABORATORY MANUAL* (*supra*).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the ASP-1 polypeptide is to be expressed for use in screening assays, generally, it is preferred that the polypeptide be produced at the surface of the cell. In this event, the cells may be harvested prior to use in the screening assay. If ASP-1 polypeptide is secreted into the medium, the medium can be recovered in order to recover and purify the polypeptide; if produced intracellularly, the cells must first be lysed before the polypeptide is recovered. ASP-1 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

Plasmid ASP1 according to the present invention was isolated and received, according to the Budapest Treaty on the international recognition of the deposit of microorganisms for the purposes of patent procedure, at the American Type Culture Collection, 10801 University Blvd, Manassas, VA 20110-2209 USA, the following ATCC Number : ATCC 209800. The deposit was received April 22, 1998.

### Diagnostic Assays according to the present invention

This invention also relates to the use of ASP-1 polynucleotides for use as diagnostic reagents. Detection of a mutated form of ASP-1 gene associated with a dysfunction will provide a diagnostic tool that can add to or define a diagnosis of a disease or susceptibility to a disease which results from under-expression, over-expression or altered expression of ASP-1. Individuals carrying mutations in the ASP-1 gene may be detected at the DNA level by a variety of techniques.

The invention relates to a diagnostic reagent for the detection of a mutated form of ASP-1 gene associated with a susceptibility to a disease containing either:
- ASP-1 polynucleotides such as described above or a variant thereof or a fragment thereof, or
- oligonucleotide probes comprising ASP-1 complementary sequences.

The probes generally will comprise at least 15 nucleotides. Preferably, such probes will have at least 30 nucleotides and may have at least 50 nucleotides.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled ASP-1 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising ASP-1 nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee et al., Science, Vol 274, pp 610-613 (1996)).

The diagnostic assays offer a process for diagnosing or determining a susceptibility to obesity, cardiovascular disorders such as hypertension and arteriosclerosis, diabetes, hypo- and hyperglycemia and anorexia through detection of mutation in the ASP-1 gene by the methods described.

In addition, obesity, cardiovascular disorders such as hypertension and arteriosclerosis, diabetes, hypo- and hyperglycemia and anorexia, can be diagnosed by methods comprising determining from a sample derived from a subject an abnormally decreased or increased level of ASP-1 polypeptide or ASP-1 mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an ASP-1 polypeptide, in a sample derived from a host are well-known to those of skill in the art. Such assay methods include radioimmunoassays, competitive-binding assays, Western Blot analysis and ELISA assays.

Therefore the present invention also provides a process for diagnosing a disease or a susceptibility to a disease in a subject related to expression or activity of ASP-1 polypeptide of the present invention in a subject comprising :
(a) determining the presence or absence of a mutation in the nucleotide sequence encoding said ASP-1 polypeptide in the genome of said subject; and/or
(b) analyzing for the presence or amount of the ASP-1 polypeptide expression in a sample derived from said subject.

The invention therefore relates also to a kit of diagnosing a disease or a susceptibility to a disease in a subject related to expression or activity of ASP-1 polypeptide in a subject containing at least:
a) a diagnostic reagent comprising an antibody specific to ASP-1 polypeptide or a fragment thereof, or
b) a diagnostic reagent comprising a fragment of ASP-1 polypeptide, or
c) a diagnostic reagent comprising ASP-1 gene polynucleotide or a probe complementary to ASP-1 gene, and
d) a mean suitable for the detection of a signal indicating the presence of a reaction between a diagnostic reagent in a), b) or c) respectively with an ASP-1 polypeptide or an antibody specific to ASP-1 polypeptide or a ASP-1 gene.

Such means include, but are not limited to, radioimmuno assay, competitive binding assays, ELISA assay.

### Chromosome Assays according to the present invention

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

The differences in the cDNA or genomic sequence between affected and unaffected individuals can also be determined. If a mutation is observed in some or all of the affected individuals but not in any normal individuals, then the mutation is likely to be the causative agent of the disease.

### Antibodies according to the present Invention

The polypeptides of the invention or their fragments or analogs thereof, or cells expressing them can also be used as immunogens to produce antibodies immunospecific for the ASP-1 polypeptides. The term "immunospecific" means that the antibodies have substantially greater affinity for the polypeptides of the invention than their affinity for other related polypeptides in the prior art.

Therefore the present invention also provides an antibody immunospecific for a ASP-1 polypeptide comprising an amino acid sequence which is at least 80% identical to the amino acid sequence of SEQ ID NO:2 over its entire length and / or which comprises the amino acid sequence of SEQ ID NO:2.

Antibodies generated against the ASP-1 polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al., Immunology Today* (1983) 4:72) and the EBV-hybridoma technique (Cole *et al.,* MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purity the polypeptides by affinity chromatography.

Antibodies against ASP-1 polypeptides may also be employed to treat obesity, cardiovascular disorders such as hypertension and arteriosclerosis, diabetes, hypo- and hyperglycemia and anorexia, among others.

### Vaccines or pharmaceutical compositions according to the present Invention

Another aspect of the invention relates to a method for inducing an immunological response in a mammal which comprises inoculating the mammal with ASP-1 polypeptide, or a fragment thereof, adequate to produce antibody and/or T cell immune response to protect said animal from obesity, cardiovascular disorders such as hypertension and arteriosclerosis, diabetes, hypo- and hyperglycemia and anorexia, among others. Yet another aspect of the invention relates to a method of inducing immunological response in a mammal which comprises, delivering ASP-1 polypeptide via a vector directing expression of ASP-1 polynucleotide *in vivo* in order to induce such an immunological response to produce antibody to protect said animal from diseases.

Further aspect of the invention relates to an immunological/vaccine formulation (composition) for the protection of a subject from diseases resulting from ASP-1 metabolism disorders which, when introduced into a mammalian host, induces an immunological response in that mammal to a ASP-1 polypeptide wherein the composition comprises a ASP-1 polypeptide or fragment thereof or ASP-1 polynucleotide.

Such a vaccine according to the present invention may be used for the treatment of a subject in need of enhanced activity or expression of ASP-1 polypeptide of the present invention and comprises:
(a) a therapeutically effective amount of an agonist to said polypeptide ; and/or
(b) a polynucleotide of the present invention in a form so as to effect production of said polypeptide activity *in vivo.*

The vaccine formulation may further comprise a suitable carrier Since ASP-1 polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain antioxidants, buffers, bacteriostats and solutes which render the formulation isotonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

Alternatively, another such vaccine according to the present invention may be used for the treatment of a subject having need to inhibit activity or expression of ASP-1 polypeptide of the present invention and comprises:
(a) a therapeutically effective amount of an antagonist to said polypeptide ; and/or
(b) a nucleic acid molecule that inhibits the expression of the nucleotide sequence encoding said polypeptide ; and/or
(c) a therapeutically effective amount of a polypeptide that competes with said polypeptide for its ligand, substrate, or receptor.

### Screening Assays according to the present Invention

The ASP-1 polypeptide of the present invention may be employed in a screening process for compounds which activate (agonists) or inhibit activation of (antagonists, or otherwise called inhibitors) the ASP-1 polypeptide of the present invention. Thus, polypeptides of the invention may also be used to assess identify agonist or antagonists from, for example, cells, cell-free preparations, chemical libraries, and natural product mixtures. These agonists or antagonists may be natural substrates, ligands, receptors, etc., as the case may be, of the polypeptide of the present invention; or may be structural or functional mimetics of the polypeptide of the present invention. See Coligan *et al., Current Protocols in Immunology* 1(2):Chapter 5 (1991).

ASP-1 polypeptides are responsible for many biological functions, including many pathologies. Accordingly, it is desirous to find compounds and drugs which stimulate ASP-1 polypeptide on the one hand and which can inhibit the function of ASP-1 polypeptide on the other hand. In general, agonists are employed for therapeutic and prophylactic purposes for such conditions as obesity, cardiovascular disorders such as hypertension and arteriosclerosis, diabetes, hypo- and hyperglycemia and anorexia. Antagonists may be employed for a variety of therapeutic and prophylactic purposes for such conditions as obesity, cardiovascular disorders such as hypertension and arteriosclerosis, diabetes, hypo- and hyperglycemia and anorexia.

In general, such screening procedures may involve using appropriate cells which express the ASP-1 polypeptide or respond to ASP-1 polypeptide of the present invention. Such cells include cells from mammals, yeast, *Drosophila* or *E. coli.* Cells which express the ASP-1 polypeptide (or cell membrane containing the expressed polypeptide) or respond to ASP-1 polypeptide are then contacted with a test compound to observe binding, or stimulation or inhibition of a functional response. The ability of the cells which were contacted with the candidate compounds is compared with the same cells which were not contacted for ASP-1 activity.

Thus the present invention provides a method for identifying compounds which inhibit (antagonize) or agonize the ASP-1 polypeptide of the present invention which comprises :
(a) contacting a candidate compound with cells which express the ASP-1 polypeptide (or cell membrane expressing ASP-1 polypeptide) or respond to ASP-1 polypeptide; and
(b) observing the binding, or stimulation or inhibition of a functional response ; or comparing the ability of the cells (or cell membrane) which were contacted with the candidate compounds with the same cells which were not contacted for ASP-1 polypeptide activity.

In a further aspect, the present invention provides an agonist and / or an antagonist identified by the hereinabove method.

### 〈〈ScreeningTechnique〉〉 according to the present invention

The assays may simply test binding of a candidate compound wherein adherence to the cells bearing the ASP-1 polypeptide is detected by means of a label directly or indirectly associated with the candidate compound or in an assay involving competition with a labeled competitor. Further, these assays may test whether the candidate compound results in a signal generated by activation of the ASP-1 polypeptide, using detection systems appropriate to the cells bearing the ASP-1 polypeptide. Inhibitors of activation are generally assayed in the presence of a known agonist and the effect on activation by the agonist by the presence of the candidate compound is observed. Standard methods for conducting such screening assays are well understood in the art.

Examples of potential ASP-1 polypeptide antagonists include antibodies or, in some cases, oligonucleotides or proteins which are closely related to the ligands, substrates, receptors, etc., as the case may be, of the ASP-1 polypeptide, e.g., a fragment of the ligands, substrates, receptors, or small molecules which bind to the polypeptide of the present invention but do not elicit a response, so that the activity of the polypeptide is prevented.

### Prophylactic and Therapeutic Methods according to the present Invention

This invention provides methods of treating an abnormal conditions related to both an excess of and insufficient amounts of ASP-1 polypeptide activity.

If the activity of ASP-1 polypeptide is in excess, several approaches are available. One approach comprises administering to a subject an inhibitor compound (antagonist) as hereinabove described along with a pharmaceutically acceptable carrier in an amount effective to inhibit activation by blocking binding of ligands to the ASP-1 polypeptide, or by inhibiting a second signal, and thereby alleviating the abnormal condition.

In another approach, soluble forms of ASP-1 polypeptides still capable of binding the ligand in competition with endogenous ASP-1 polypeptide may be administered. Typical embodiments of such competitors comprise fragments of the ASP-1 polypeptide.

In still another approach, expression of the gene encoding endogenous ASP-1 polypeptide can be inhibited using expression blocking techniques. Known such techniques involve the use of antisense sequences, either internally generated or separately administered. See, for example, O'Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al., Nucleic Acids Res* (1979) 6:3073; Cooney *et al., Science* (1988) 241:456; Dervan *et al., Science* (1991) 251:1360. These oligomers can be administered *per se* or the relevant oligomers can be expressed *in vivo*.

For treating abnormal conditions related to an under-expression of ASP-1 and its activity, several approaches are also available. One approach comprises administering to a subject a therapeutically effective amount of a compound which activates ASP-1 polypeptide, i.e., an agonist as described above, in combination with a pharmaceutically acceptable carrier, to thereby alleviate the abnormal condition. Alternatively, gene therapy may be employed to effect the endogenous production of ASP-1 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo*. For overview of gene therapy, see Chapter 20, *Gene Therapy and other Molecular Genetic-based Therapeutic Approaches*, (and references cited therein) in Human Molecular Genetics, T Strachan and A P Read, BIOS Scientific Publishers Ltd (1996).

### Formulation and Administration according to the present Invention

Peptides, such as the soluble form of ASP-1 polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide ex vivo, and for example, by the use of a retroviral plasmid vector. The cells are then introduced into the subject.

## Claims

1. An isolated human polynucleotide comprising:
a) a nucleotide sequence that has at least 80% identity to a nucleotide sequence encoding the ASP-1 polypeptide of SEQ ID NO:2 over its entire length, or
b) a nucleotide sequence complementary to said nucleotide sequence, or
c) fragments of a) or b) encoding biologically active polypeptides.

2. The polynucleotide of claim 1 which is DNA or RNA.

3. The human polynucleotide of claim 1 wherein said nucleotide sequence is at least 80% identical to that contained in SEQ ID NO:1.

4. The polynucleotide of claim 3 wherein said nucleotide sequence comprises the ASP-1 polypeptide encoding sequence contained in SEQ ID NO:1.

5. The polynucleotide of claim 3 which is polynucleotide of SEQ ID NO: 1.

6. Probes for the isolation or amplification of cDNA or genomic DNA consisting of a polynucleotide according to claim 1 or a fragment thereof.

7. Probe according to claim 6 comprising at least 30 nucleotides, preferably between 30 and 50 nucleotides.

8. Method for screening an appropriate library comprising the step of hybridization under stringent condition with a probe according to claim 6 or 7.

9. A DNA or RNA molecule comprising an expression system, or vector, wherein said expression system is capable of producing a ASP-1 polypeptide comprising an amino acid sequence, which has at least 80% identity with the polypeptide of SEQ ID NO:2 when said expression system is present in a compatible host cell.

10. A host cell comprising the expression system of claim 9.

11. A process for producing a ASP-1 polypeptide comprising culturing a host of claim 10 under conditions sufficient for the production of said polypeptide and recovering the polypeptide from the culture.

12. A process for producing a cell which produces a ASP-1 polypeptide thereof comprising transforming or transfecting a host cell with the expression system of claim 9 such that the host cell, under appropriate culture conditions, produces a ASP-1 polypeptide.

13. A ASP-1 polypeptide comprising an amino acid sequence which is at least 80% identical to the amino acid sequence of SEQ ID NO:2 over its entire length.

14. The polypeptide of claim 13 which comprises the amino acid sequence of SEQ ID NO:2.

15. Fragments of ASP-1 polypeptide according to claim 13 or 14 characterized in that:
a) they retain the biological activity of ASP-1 including antigenic activity or,
b) they have the sequence of ASP-1 polypeptides of claim 13 or 14 deleted of a continuous series of residues including the amino terminus or the carboxyl terminus or both or,
c) they are a variant of a) or b) by conservative amino-acid substitutions.

16. An antibody immunospecific for the ASP-1 polypeptide or fragments thereof of claims 13 to 15.

17. Fragments of ASP-1 polypeptides recognized by an antibody according to claim 16.

18. Diagnostic reagent for the detection of the presence or amount of ASP-1 polypeptide comprising an antibody according to claim 16.

19. Diagnostic reagent for the detection of the presence or amount of an antibody according to claim 13 comprising a fragment according to claim 17.

20. Diagnostic reagent for the detection of a mutated form of ASP-1 gene associated with a susceptibility to a disease containing either:
a) ASP-1 polynucleotides according to anyone of claims 1 to 7, or
b) oligonucleotide probes comprising ASP-1 complementary sequences or fragment thereof.

21. A kit for diagnosing a disease or a susceptibility to a disease in a subject related to expression or activity of ASP-1 polypeptide containing at least:
a) a diagnostic reagent according to claim 18, or
b) a diagnostic reagent according to claim 19, or
c) a diagnostic reagent according to claim 20, and
d) a mean suitable for the detection of a signal indicating the presence of a reaction between a diagnostic reagent in a), b) or c) respectively with an ASP-1 polypeptide or an antibody specific to ASP-1 polypeptide or a ASP-1 gene.

22. A method for identifying compounds which inhibit (antagonize) or agonize the ASP-1 polypeptide of claim 13 which comprises:
(a) contacting a candidate compound with cells which express the ASP-1 polypeptide (or cell membrane expressing ASP-1 polypeptide) or respond to ASP-1 polypeptide ; and
(b) observing the binding, or stimulation or inhibition of a functional response ; or comparing the ability of the cells (or cell membrane) which were contacted with the candidate compounds with the same cells which were not contacted for ASP-1 polypeptide activity.

23. An agonist identified by the method of claim 22.

24. An antagonist identified by the method of claim 22.

25. Plasmid ASP1 ATOC N° : 209800.

26. Vaccine for the protection of a subject from diseases resulting from ASP-1 metabolism disorders, containing as an active principle an ASP-1 polypeptide according to anyone of claims 13 to 15 or 17, or a ASP-1 polynucleotide according to anyone of claims 1 to 5.

27. Therapeutic composition for the treatment of a subject having need to inhibit activity or expression of ASP-1 polypeptide of claim 13 or 14 comprising :
(a) a therapeutically effective amount of an antagonist to said polypeptide; and/or
(b) a nucleic acid molecule that inhibits the expression of the nucleotide sequence encoding said polypeptide ; and/or
(c) a therapeutically effective amount of a polypeptide that competes with said polypeptide for its ligand, substrate, or receptor.

28. Therapeutic composition for the treatment of a subject in need of enhanced activity or expression of ASP-1 polypeptide of claim 13 or 14 comprising :
(a) a therapeutically effective amount of an agonist to said polypeptide ; and/or
(b) a polynucleotide of any one of claims 1 to 5 in a form so as to effect production of said polypeptide activity *in vivo*.
